# EUROPEAN PATENT SPECIFICATION

(11) **EP 0 707 474 B1**
(45) Date of publication and mention of the grant of the patent: **16.08.2000**
(21) Application number: 94918978.1
(22) Date of filing: 30.06.1994
(51) Int. Cl.: A61K 9/16, A61K 49/00, A61L 27/00

(54) **MEDICAL USE OF ORGANIC AEROGELS AND BIODEGRADABLE ORGANIC AEROGELS**
MEDIZINISCHE VERWENDUNG ORGANISCHER AEROGELE SOWIE BIOLOGISCH ABBAUBARE ORGANISCHE AEROGELE
UTILISATION MEDICALE D'AEROGELS ORGANIQUES ET D'AEROGELS ORGANIQUES BIODEGRADABLES

(30) Priority: 30.06.1993 GB 9313501
(43) Date of publication of application: 24.04.1996
(73) Proprietor: NYCOMED IMAGING AS, 0401 Oslo 4 (NO)
(72) Inventor: BERG, Arne, N-1300 Sandvika (NO); DROEGE, Michael, William, Livermore, CA 94550 (US); FELLMANN, Jere, Douglas, Livermore, CA 94550 (US); KLAVENESS, Jo, N-1166 Oslo (NO); RONGVED, Pal, N-1450 Nesoddtangen (NO)
(74) Representative: Marsden, John Christopher
(86) International application number: GB9401421
(87) International publication number: WO9501165

(56) References cited:
- WO-A-91/09079
- US-A- 4 818 542
- US-A- 4 873 218
- US-A- 5 081 163

## Description

This invention is concerned with the medical use of physiologically acceptable organic aerogels, including biodegradable organic aerogels and carbonised organic aerogels.

The term aerogel is used hereinafter to describe solid materials characterised by high porosity, of the order of 80-99.8%, and by high surface area. The pores typically have sizes of from about 10 nm to values in the micrometre range. Aerogels are most commonly manufactured by supercritical drying of gels, and may be contrasted with xerogels and cryogels, which are respectively obtained by normal evaporative drying and by freeze drying of gels and which normally exhibit maximum porosities of about 50%.

Aerogels may also be contrasted with porous biodegradable polymer sponges such as are described in WO-A-9109079, which are prepared by contacting a non-gelled polymer with a supercritical fluid in a chamber and subsequently sharply reducing the pressure in the chamber, thereby expanding the polymer structure to form a porous sponge. Because the polymer molecules are randomly arranged and are disrupted during the pressure reduction step, the resulting sponges will lack the structural integrity of aerogels prepared by supercritical drying of conformationally stable polymer gels. This will inevitably limit the maximum porosity which may be achieved, and a maximum obtainable porosity of 74% is quoted.

Silica-based aerogels were first described by S.S. Kistler in Nature 127 (1931), p. 741. Kistler's prediction that a wide range of other substrates, both inorganic and organic, could also be useful in the manufacture of aerogels has proved correct and numerous publications regarding such materials have issued, particularly in the last 10-15 years, involving such diverse applications as use in acoustics technology, as insulators for solar cells, as catalysts, as ion exchange and chromatographic separation media, in Cerenkov detectors and for inertial confinement fusion targets.

Silicon-based aerogels formed by hydrolysis/ condensation of metal alkoxides are described by, for example, L.W. Hrubesh et al. in Chemical Processing of Advanced Materials (1992), Chapter 2 (ISBN 0-471-54201-6); T.M. Tillotson and L.W. Hrubesh in J. Non-Cryst. Solid 145 (1992), p. 44; Aerogels ed J. Fricke (Springer-Verlag, New York, 1986); S.J. Teichner et al. in Adv. Coll. Interf. Sci. 5. (1976), p. 245; C.J. Brinker et al. in J. Non-Cryst. Solids 63 (1984), p. 45; and in the references therein. The production of transparent insulating silica aerogel arrays using e.g. liquid carbon dioxide as the solvent to be removed in the supercritical drying stage is described in US-A-4610863 (U.S. Department of Energy). The manufacture of hollow aerogel spheres, particularly of silica, e.g. for use in controlling nuclear fusion, for very high surface area catalyst supports and as insulators, is described in published application US-A-7620123 (U.S. Department of Energy). A range of metal oxide aerogels and their production from corresponding metal alkoxides by a two step reaction process is described in WO-A-92033378.

The use of inorganic aerogels as pyro-optic sensors for IR thermal imaging is described in US-A-4994672 (Pennsylvania Research). Other proposals for inorganic aerogels include use as components of aerosol therapeutic compositions containing copper powder - see US-A-4123511 (Gertrude Fischer); as components of composite foams e.g. for use as inertial confinement fusion targets - see US-A-4966919 and US-A-5037859 (U.S. Department of Energy); as a substrate for loading with tritium - see US-A-5078919 (U.S. Department of Energy); as components in dentifrice tablets containing Vitamin E - see US-A-4411885 (Barels and Ghinazzi); as components in heat releasing dentifrices - see US-A-4132771 (Schreiber and Principe); as additives in devices for controlling insect pests in food - see US-A-4927635 (Loschiavo); and as components in insecticidal aerosol compositions - see IL-A-79449 (Sano Brunos Entrepr.). Other publications relating to silica aerogels in insecticides include White et al. in J. Econ. Entomol. 82(3) (1989), p. 960; Loschiavo et al. in J. Econ. Entomol. 81(4) (1988), p. 1237; and Loschiavo et al. in J. Econ. Entomol. 81(4) (1988), p. 1231.

Organic aerogels which have been described in the literature include resorcinol-formaldehyde aerogels (US-A-4873218 - U.S. Department of Energy); melamine-formaldehyde aerogels (US-A-5086085 and US-A-5081163 - U.S. Department of Energy), and carbon foams obtained by pyrolysis of such aerogels.

Publications relating to organic aerogels include Chemical Processing of Advanced Materials (ed. L.L Hench and J.K. West), Chapter 60 - R.W. Pekala et al, p. 671; R.W. Pekala and C.T. Alviso in Mater. Res. Soc. Sym. Proc. 180 (1990), p. 791; R.W. Pekala in J. Mater. Sci. 24 (1989), p. 3221; R.W. Pekala and F.M. Kong in J. Phys. Coll. Suppl. 50(4) (1989), p.C4-33; R.W. Pekala and F.M. Kong in Polym. Prpts. 30(1) (1989), p.221; R.W. Pekala and R.L. Ward in Polym. Prpts. 31 (1) (1990), p.167; R.W. Pekala et al. in J. Non-Crystal. Solid 145 (1992), p. 90; X.Lu et al. in Science 255 (1992), p.971; Energy & Technology (January 1991) Lawrence Livermore National Laboratory; and MRS Bulletin (International Materials Research Community) 15 (12) (December 1990), pp. 19-45.

The present invention is based, inter alia, on our finding that physiologically compatible organic aerogels have a wide range of medical uses, for example as diagnostic agents, artificial tissues, organs and organ components etc., and in particular in drug delivery systems. More especially we have found that organic aerogels are particularly stable and retain their integrity in vivo (although, as described hereinafter, the constituent polymer may be selected so as to be biodegradable over a chosen period of time). This may be contrasted with the behaviour of inorganic (e.g. silica-based) aerogels which, particularly when prepared by supercritical removal of carbon dioxide, tend to be hydrophilic so that their structure is potentially unstable in the presence of moisture or body fluids such as blood.

Thus, according to one aspect of the present invention, there is provided a physiologically acceptable organic aerogel for use in medicine, for example in the manufacture of medicaments, healing aids and/or as prostheses, e.g. for human or veterinary purposes, said aerogel having a porosity of 80-99.8% and being obtainable by supercritically drying a polymer gel.

It will be appreciated that in order to be physiologically acceptable the organic aerogels must comprise materials which are non-toxic, non-carcinogenic and sterilisable, which do not cause inflammation or other clinically significant changes in body tissues and/or fluids, and which when subject to degradation in vivo, e.g. as a result of enzymatic or hydrolytic action, yield physiologically acceptable metabolites.

In principle organic aerogels used in accordance with the invention may comprise any polymer which fulfils this requirement of physiological acceptability and which is capable of gel formation, e.g. as a result of the presence or subsequent introduction of an appropriate proportion of crosslinking groups or as a result of being obtained by polymerisation of one or more appropriate multifunctional monomers; copolymers and crosslinked polymers obtained from two or more appropriate multifunctional monomers may advantageously be used. Carbonised aerogels obtained by pyrolysis of such polymeric aerogels may also be used in accordance with the invention.

Examples of physiologically acceptable polymers for which in vivo medical applications have been described or proposed, and which may therefore be useful in aerogel form in accordance with the invention, are given in Polymers in Medicine (ed. R. Ottenbrite and E. Chiellini) - Technomic, Lancaster, USA (1992); Biodegradable Polymers and Plastics (ed. M. Vert, J. Feyen, A. Albertson, G. Scott and E. Chiellini) - The Royal Society of Chemistry, Cambridge (1992); Blood Compatible Materials and Devices (ed. C.P. Sharma and M. Szycher) - Technomic, Lancaster, USA (1992) and references therein; US-A-4643715 (Terumo Kabushiki Kaisha); US-A-4634447 (Terumo Kabushiki Kaisha); US-A-4588407 (Terumo Kabushiki Kaisha); JP-A-92062742 (Toray); JP-A-4168297 (Advance KK); JP-A-4004868 (Toray); JP-A-2238007 (Oki Electric Ind.); JP-A-90006538 (Terumo Corp.); JP-A-63079667 (Senoo); JP-A-88007788 (Terumo Corp.); US-A-4277595 (Bausch & Lomb); JP-A-89297103 (Asahi Chemical Industry); JP-A-8736323 (Mitsubishi Ryon); Applied Polymer Science 2 (1985), p.535; F.J. Schoen in ASAIO Trans. 37 (1991), p.44; D. Blockmans and J. Vemylen in Int. J. Artif. Organs 13 (1990), p.723; J.A. Schmitt-Fournier et al. in Rev. Chir. Orthop. 74 Suppl. 2 (1988), p.218; various authors in Biomater. Artif. Cells Artif. Organs 16 (1988), p. 829; J.J. Rosen in ASAIO Trans 34 (1988), p.163; F.J. Schoen et al. in ASAIO Trans 33 (1987), p. 824; L.W. Henderson and D. Chenoweth in Blood Purif. 5 (1987), p. 100; D.E. Taylor and J.E. Penhallow in Biomaterials 7 (1986), p.277; H. Mori and K. Esato in Iydenshi To Seitai Kogaku 23 (1985), p.203; W.S. Pierce in Trans. Am. Soc. Artif. Intern. Organs 30 (1984), p. 721; K. Imachi and K. Atsumi in Iyodenshi To Seitai Kogaku 21 (1983), p. 381; I. Fujimasa in Iyodenshi To Seitai Kogaku 21 (1983), p.372; T. Akaike in Iyodenshi To Seitai Kogaku 21 (1983), p. 337; R. Thull in Med. Prog. Technol. 9 (1982), p. 119; and H.A. McAdoo et al. in Quintessence Int. 12 565 (1981).

It will be preferred in many embodiments of the invention to employ organic aerogels which are biodegradable, i.e. which comprise polymers containing hydrolytically and/or enzymatically cleavable bonds in the main chain and/or in any crosslinking units. It will be appreciated that biodegradability is particularly desirable for materials such as diagnostic and therapeutic agents which are to be administered parenterally, in order to facilitate their ultimate elimination from the body.

Representative hydrolytically/enzymatically cleavable bonds which may be present in such biodegradable organic aerogels include ester, carbonate, orthoester, anhydride, acetal and amide linkages. Representative biodegradable polymer components of these aerogels thus include polyesters and copolymers thereof; polyorthoesters and copolymers thereof; non-peptide polyamides; peptide polyamides; polyanhydrides; polyphosphazenes; polyiminocarbonates; polycyanoacrylates; naturally occurring polymers such as hyaluronic acid, chitosan, chitin, collagen, gelatin, albumin and starch; and other synthetic, semisynthetic and naturally occurring materials.

One preferred class of biodegradable polymers components comprises polymers containing methylene diester groups of the formula (I)

⁅ CO-O-C(R¹R²) -O-CO ⁆ (I)

(where R¹ and R² each represent a hydrogen atom or a carbon-attached monovalent organic group or R¹ and R² together form a carbon-attached divalent organic group). Such units are particularly rapidly degraded by common esterase enzymes but are stable in the absence of enzymes. They may be attached not only to carbon-attached organic groups as in simple carboxylate esters but also to -O- atoms as in carbonate esters - see e.g. WO-A-9204392 and WO-A-9308070.

Organic aerogels useful in accordance with the invention are preparable by supercritical drying of corresponding polymer gels. Such gels may be obtained by, for example, sol-gel polymerisation of an appropriate multifunctional monomer in analogous manner to the sol-gel reactions of metal alkoxides etc. used in the preparation of inorganic aerogels.

Alternatively the gels may be obtained by water-swelling appropriately crosslinked derivatives of polymers which are water-soluble in non-crosslinked form, such as polyvinyl alcohol, polyacrylic acid, polymethacrylic acid, polyhydroxyalkyl acrylates and methacrylates such as poly(2-hydroxyethyl acrylate), polysaccharides, polyesters, polyethers such as polyoxyethylenes and polyoxypropylenes, polyacrylamides and polymethacrylamides such as poly(N-hydroxyalkyl) acrylamides and methacrylamides, polyamides, polyurethanes and epoxy polymers. It will be appreciated that the water-swelling and any necessary polymerisation and/or crosslinking reactions may if desired be effected simultaneously, e.g. using techniques standard in the polymer art.

In the supercritical drying stage the solvent of choice for the gel is liquid carbon dioxide, which has its critical point at 31°C and 7.4 MPa, thus permitting supercritical drying under mild temperature conditions which should minimise any tendency to degradation of the polymer. Other solvents with low critical temperature which may be useful for this purpose include methanol, nitrous oxide, Freon 13 (CClF₃), Freon 23 (CHF₃) and Freon 116 (CF₃-CF₃).

Since liquid carbon dioxide is not miscible with water, it will be necessary where a water-based polymer gel (i.e. an aquagel) is initially formed to exchange the water with an organic solvent such as acetone which is miscible with both water and liquid carbon dioxide. Exchange of this organic solvent by liquid carbon dioxide and subsequent supercritical extraction of the carbon dioxide may be effected in a temperature-controlled pressure vessel such as an autoclave.

In addition to being used in medicine in accordance with the invention, organic aerogels may also be used as intermediates in the manufacture of appropriate end products by suitable forms of treatment. Thus, for example, organic aerogels may be treated with aqueous media to generate aquagels or with heat to generate carbonised aerogels; such carbonised aerogels may, for example, be formed by pyrolysing materials such as resorcinol-formaldehyde aerogels, e.g. at 1050°C under an inert atmosphere.

It will be appreciated that reaction parameters such as pH, temperature and reagent concentrations during sol-gel polymerisation and the degree of crosslinking and swelling will all affect the properties of aerogels obtained from polymer gels, in particular their density, degree of porosity and pore size.

One important medical use of physiologically acceptable organic aerogels in accordance with the invention is as diagnostic agents. Such agents constitute a further feature of the present invention.

It is well known that ultrasonic imaging comprises a potentially valuable diagnostic tool, for example in studies of the vascular system, particularly in cardiography, and of tissue microvasculature. A variety of contrast agents has been proposed to enhance the acoustic images so obtained, including suspensions of solid particles, emulsified liquid droplets, gas bubbles and encapsulated gases or liquids. It is generally accepted that low density contrast agents which are easily compressible are particularly efficient in terms of the acoustic backscatter they generate, and considerable interest has therefore been shown in the preparation of gas-containing and gas-generating systems.

Initial studies involving free gas bubbles generated in vivo by intracardiac injection of physiologically acceptable substances have demonstrated the potential efficiency of such bubbles as contrast agents in echocardiography; such techniques are severely limited in practice, however, by the short lifetime of the free bubbles. Interest has accordingly been shown in methods of stabilising gas bubbles for echocardiography and other ultrasonic studies, for example using emulsifiers, oils, thickeners or sugars, or by entraining or encapsulating the gas or a precursor therefor in a variety of polymer systems, e.g. as porous gas-containing polymer microparticles or as gas "microballoons" encapsulated by polymer coatings.

Thus, for example, WO-A-8002365 discloses the use of gelatin encapsulated gas microbubbles for enhancing ultrasonic images. Such microbubbles do not, however, exhibit adequate stability at the dimensions preferred for use in echocardiography (1-10 µm) in view of the extreme thinness of the encapsulating coating.

US-A-4774958 discloses the use of microbubble dispersions stabilised by encapsulation in denatured protein, e.g. human serum albumin. Such systems permit the production of microbubble systems having a size of e.g. 2-5 µm but still do not permit efficient visualisation of the left heart and myocardium. The use of such protein-derived agents may also create problems with regard to potential allergenic reactions.

EP-A-0327490 discloses, inter alia, ultrasonic contrast agents comprising a microparticulate synthetic biodegradable polymer containing a gas or volatile fluid (i.e. having a boiling point below 60°C) in free or bonded form. Representative synthetic biodegradable polymers include polyesters of hydroxy carbonic acids, polyalkyl cyanoacrylates, polyamino acids, polyamides, polyacrylated saccharides and polyorthoesters.

Similar biodegradable microparticulate polymers, based on polymerised aldehydes, are described in EP-A-0441468, while systems based on microparticulate poly (amino acid) - poly (cyclic imide) derivatives are described in EP-A-0458079.

EP-A-0458745 discloses air or gas-filled microballoons in which the encapsulating material is a deformable and resilient interfacially deposited polymer which is preferably biodegradable, examples including polysaccharides, polyamino acids, polylactides, polyglycolides, lactide/lactone copolymers, polypeptides, proteins, polyorthoesters, polydioxanone, poly-β-aminoketones, polyphosphazenes, polyanhydrides and poly (alkyl cyanoacrylates). The microballoons are normally prepared by emulsion techniques leading to deposition of the polymer around droplets of a volatile liquid which is subsequently evaporated.

In WO-A-9112823 there are described ultrasound contrast agents comprising gas- or vapour-filled polymer microcapsules; preferred polymers include insolubilised proteins such as denatured albumin. The microcapsules may be prepared by forming a protein shell around a solid or liquid core (e.g. by methods using simple or complex coacervation, double emulsion or minimisation of solubility at isoelectric point), hardening the shell (e.g. by chemical or heat treatment), and removing the core (e.g. by sublimation or evaporation). The use of double emulsion techniques will provide microcapsules having a honeycomb structure with multiple gas- or vapour-filled chambers.

Gas-containing contrast media are also known to be effective in magnetic resonance (MR) imaging, e.g. as susceptibility contrast agents which will act to reduce MR signal intensity. Oxygen-containing contrast media also represent potentially useful paramagnetic MR contrast agents.

Furthermore, in the field of X-ray imaging it has been observed that gases such as carbon dioxide may be used as negative oral contrast agents.

It is generally acknowledged that polymer-based contrast agents should desirably be biodegradable in order to facilitate their ultimate elimination from or absorption by the test subject. Little attention has been given, however, to the specific design of polymers to maximise this objective, reliance generally being made on the inherent, albeit slow, biodegradability of polymers such as polyesters, polyanhydrides, polycarbonates, polyamides and polyurethanes which principally results from the susceptibility of ester, amide or urethane groups therein to enzymic hydrolysis.

Any of the polymers described above for use as contrast agents or any other physiologically acceptable and preferably also biodegradable polymer may be used to produce aerogel diagnostic agents in accordance with the invention, the principal requirement being that the polymer is gel-forming or can be adapted to be gel-forming, e.g. by the introduction of crosslinking groups.

Aerogels are characterised by low densities and low sound transmission velocities and organic aerogels therefore have important applications as contrast agents in ultrasound imaging. Such contrast agents combine the desiderata of very high and long lasting contrast efficacy, good storage stability and minimal toxicity.

For use as ultrasound contrast agents the organic aerogels may, for example, be formulated as microparticles. For applications such as echocardiography, in order to permit free passage through the pulmonary system, such microparticles conveniently have sizes not exceeding 10 µm, e.g. not exceeding 7 µm, preferably not exceeding 5 µm. Large microparticles, e.g. with sizes of up to 500 µm, may however be useful in other applications such as gastrointestinal imaging or investigations of the Fallopian tubes. The microparticles may be made by, for example, conventional micronisation techniques such as milling, e.g. ball-milling, of macroscopic aerogel material, or may be prepared e.g. using emulsion techniques.

Any biocompatible gas may be present in the pores of organic aerogels used as ultrasound contrast agents, for example air, nitrogen, oxygen, hydrogen, nitrous oxide, carbon dioxide, helium, argon, sulphur hexafluoride and low molecular weight optionally fluorinated hydrocarbons such as methane, ethane, propane, acetylene or carbon tetrafluoride. The term "gas" as used in this context includes any substance which is in gaseous form at the normal human body temperature of 37°C. The presence in the aerogels of lipophilic gases having low aqueous solubility, for example fluorinated alkanes such as perfluorobutane or perfluoropentane, may have a stabilising effect on aerogels comprising water-soluble or water-sensitive materials, since such gases will have a lower tendency than air to dissolve in a surrounding aqueous medium (e.g. a formulating medium prior to administration or a body fluid such as blood after administration), thereby miminising intrusion of such a medium into the aerogel structure. Aerogels containing such lipophilic gases may also serve as gas-releasing particles, for example generating gas microbubbles which may be useful in diagnostic imaging.

Organic aerogel microparticles will also act as negative X-ray contrast agents because of their low density compared to body tissues.

Alternatively the aerogels may be adapted to exhibit a positive X-ray contrast effect by incorporation of one or more substances which have higher X-ray absorption than tissue. Such X-ray contrast agents normally contain one or more heavy elements as in, for example, compounds containing tungsten clusters and/or iodinated aromatic groups, preferably so as to contain at least three iodine atoms per molecule. The X-ray contrast agent may be physically incorporated into the aerogel structure, in which case non-ionic X-ray contrast agents are preferred, or may be chemically linked thereto, e.g. by covalent bonding. Particularly preferred X-ray contrast agents for use in accordance with this embodiment of the invention include non-ionic tri-iodinated compounds such as iohexol and iopamidol, and non-ionic dimeric compounds such as iodixanol.

Aerogel X-ray contrast agents may be used in such applications as liver imaging, bronchography and cardiovascular imaging.

Organic aerogel microparticles are additionally useful as contrast agents in magnetic resonance imaging (MRI), exhibiting a susceptibility contrast effect when adminstered at comparatively high doses. The aerogels may also be adapted to incorporate one or more MR contrast agents. As with the X-ray contrast agents such MR contrast agents may be physically incorporated into the aerogel structure or may be chemically linked thereto.

Useful MR contrast agents include paramagnetic compounds such as transition metal chelates and lanthamide chelates, examples including MnEDTA, MnDPDP, GdDTPA, GdDTPA-BMA, GdDOTA, GdHPD03A, GdBOPTA, DyDTPA, DyDTPA-BMA and other physiologically acceptable acyclic or macrocyclic chelates. Other possible MR contrast agents include fluoro compounds and stable free radicals.

Aerogel MR contrast agents may be used in such applications as blood pool imaging and liver imaging.

Aerogel contrast agents according to the invention may be stored and transported in dry form, in which condition they will normally be stable indefinitely, being mixed with an appropriate liquid carrier (e.g. sterile water for injection, physiological saline or phosphate buffer) prior to administration. In this way the concentration of the injected or otherwise administered contrast agent may be varied at will. They may also be stored ready for administration as suspensions in such carriers and will again exhibit good storage stability, particularly if the polymer material is relatively hydrophobic.

The organic aerogels may be sterilised after manufacture by, for example, standard methods such as gamma irradiation, ultraviolet irradiation, electron beam irradiation, steam treatment, or treatment with ethylene oxide or with liquid chemical sterilants.

A further medical use of physiologically acceptable organic aerogels in accordance with the invention is as drug delivery systems or components of such systems. Such delivery systems, which are advantageous in that they permit high loading of active ingredients to give systems which are physiologically acceptable, of low toxicity and high efficacy while exhibiting favourable pharmacokinetic properties together with high biodegradability, constitute a further feature of the invention.

The therapeutic drug may be physically incorporated into the aerogel structure or may be chemically linked thereto, if desired through a spacer arm. It will be appreciated that such chemical linkages should normally be cleavable, e.g. by hydrolytic and/or enzymatic action, to permit release of the active ingredient from the aerogel polymer matrix. Thus, for example, carboxyl group-containing medicaments may conveniently be chemically linked to hydroxyl group-containing aerogel structures through readily hydrolysable ester groups.

Medicaments which may be used in delivery systems according to the invention include CNS agents, anti-inflammatory agents, diuretics, cardiovascular drugs, antineoplastic agents, drugs affecting gastrointestinal functions, antimicrobial and antiparasitic agents, hormones, hormone antagonists, and drugs acting on the blood and blood-forming organs.

Organic aerogels used in or as components of drug delivery systems may, for example, be formulated as suspensions of particles for injection, e.g. in similar manner to organic aerogel contrast agents, and may, for example, be adminstered via intracutaneous, subcutaneous, intramuscular, intravascular, intracardiac, intraspinal, intra-articular and opthalmic routes. Aerosol formulations may also be advantageous, especially by virtue of the low density of the aerogels. Other possible formulations include lamellae for opthalmic administration, e.g. of agents such as pilocarpine, and units for implantation, e.g. for administration of hormones or of alcohol deterrents such as disulfiram.

Ophthalmic applications of drug delivery systems may take advantage of the optical transparency exhibited by many aerogels.

Contrast properties of the aerogel in drug delivery systems of the types described above, particularly their ultrasound contrast effect, may if desired be monitored to determine factors such as distribution and eliminations of the drug.

Physiologically acceptable organic aerogels may also be used as healing aids, prostheses, artificial tissues, implants and the like, such articles comprising a further feature of the invention. Such use of physiologically acceptable organic aerogels is rendered advantageous by virtue of their being non-toxic, non-carcinogenic, non-immunogenic, sterilisable and readily fabricable. Applications as e.g. artificial tissues and implants are particularly advantageous in that the pore size of the aerogels is such that body tissue may grow into the polymer material.

Organic aerogels may be used in accordance with this aspect of the invention as, for example, artificial skin, artificial organs and components thereof (e.g. artificial hearts and heart components such as cardiac valves), cardiac pacemakers and assist systems, membranes for insulin pumps, carriers for betacells, artificial eye components (e.g. intraocular lenses, contact lenses, vitreous humour and corneal prostheses), arterial and vascular prostheses, vascular grafts, stents, shunts, catheters, articular cartilage, blood prosthetic devices, and particles for embolisation. Where appropriate, small amounts of active drugs such as antibiotics or immunosuppressives may be incorporated into the aerogel.

Examples of these and other medical devices for use in or in contact with the body, in surgery or otherwise, are well known in the art and are described in for example, the literature references listed hereinbefore with regard to in vivo medical applications of polymers.

In certain applications it may be desirable to modify the surface of the organic aerogel, e.g. to ensure that the material is blood-compatible or to affect the absorption of protein. Thus, for example, the surface of the organic aerosol may be modified so as selectively to bind albumin from blood, e.g. by methods similar to those described for other systems in Blood Compatible Materials and Devices (ed. C.P. Sharma and M. Szycher) - Technomic, Lancaster, USA (1992); and by T. Perter et al. in J. Biol. Chem. 248 (1973), p. 2447; S.D. Riccitelli et al. in Trans. Am. Soc. Artif. Int. Organs 31 (1985), p. 250, McD.K. Horne in Thromb. Res. 37 (1985), p. 201; J.L. Brasch and T.A. Horbett (eds) in Amer. Chem. Soc. Ser. 343 - ACS Books, Washington D.C. (1987); and N.P. Ziats in Trans. Soc. Biomat. 13 (1990), p. 268.

Other methods for modifying organic aerogel surfaces include heparinization, e.g. using methods similar to those proposed for other systems by R.I. Leininger in Crit. Rev. Bioengineering 1 (1972), p. 333; S.W. Kim et al. in ASAIO J. 6 (1983), p. 76; M.W. Hatton et al. in Thromb. Haemostas. 50 (1984), p. 873; C.M. Arnander in J. Biomed. Mater. Res. 20 (1986), p. 235; N. Platé in Biomaterials 4 (1983), p. 14; C. Fougnot in Ann. Biomed. Eng. 7 (1979), p. 429; C. Fougnot in Ann. Biomed. Eng. 7 (1979), p. 441; W.E. Hennink in Trans. Am. Soc. Artif. Int. Organs 29 (1983), p. 200, and B.K. Kusserow et al. in Trans. Am. Soc. Artif. Int. Organs 17 (1971), p. 1. Other surface modifications for selective absorbtion are also possible for organic aerogels, for example by methods such as are used for other systems by M.S. Munro et al. in ASAIO J. 6 (1983), p. 65; and F. Senatore et al. in J. Biomed. Mater. Res. 20 (1986), p. 177.

The aerogel may be coated with other coatings, for example to increase the stability of the materials. Thus, for example, a bioacceptable coating may be applied to seal the pores and thus stabilize the gas content of the aerogel, e.g. so as to maintain its ultrasound contrast properties.

The following non-limitative examples serve to illustrate the invention.

### EXAMPLES 1-4 - PREPARATION OF ORGANIC AEROGELS

These preparations are performed as described in the literature (Pekala, Mayer, Kaschmitter and Kong in "Carbon Aerogels: An Update on Structure, Properties, and Applications"; Lawrence Livermore National Laboratory; UCRL-JC-114192 Preprint; July 1993).

### Example 1

### Preparation of monolithic resorcinol-formaldehyde aerogels

Resorcinol and formaldehyde (1:2 molar ratio) are mixed in sufficient deionised distilled water to produce a solution containing ≤10% w/v of reactants. Sodium carbonate is then added as a catalyst (resorcinol:catalyst molar ratio 50-400:1). The mixture is stirred to produce a homogeneous solution which is poured into glass vials, sealed and allowed to cure to a gel at 85-95°C for seven days.

After curing is completed, the cross-linked gel is removed from the glass vial and the pore fluid (water) is exchanged with acetone. The acetone-filled gel is placed in a jacketed pressure vessel (Polaron), the acetone is then exchanged with liquid carbon dioxide and the gel is supercritically dried (Tc=31°C; Pc=7.4 MPa), yielding a monolithic resorcinol-formaldehyde aerogel retaining the shape of the glass vial used in the curing stage, i.e. in the shape of a cylinder approx. 25 mm x 80 mm.

### Example 2

### Preparation of microspheres of resorcinol-formaldehyde aerogel

Resorcinol and formaldehyde (1:2 molar ratio) are mixed in sufficient deionised distilled water to produce a solution containing ≤ 10% w/v of reactants. Sodium carbonate is then added as a catalyst (resorcinol:catalyst molar ratio 50-400:1). The mixture is stirred to produce a homogeneous solution. An inverse emulsion polymerisation is used to produce the resorcinol-formaldehyde aerogel microspheres. One litre of cyclohexane is heated to 50-70°C in a glass reaction vessel. In a separate vessel, the resorcinol-formaldehyde solution (as prepared above) is partially polymerised (at 85-90°C) until approaching the gel point. Approximately 150 ml of this solution is poured into the cyclohexane with vigorous stirring. The resorcinol-formaldehyde solution disperses into spherical droplets with a size depending on the stirring rate and on the presence or absence of surfactant. After heating the inverse emulsion for 2-8 hours, the stirring is stopped and the spheres are allowed to settle. The cyclohexane is decanted from the reaction vessel and replaced with acetone. After displacement of the pore fluid with acetone the acetone is exchanged with liquid carbon dioxide and the microspheres are supercritically dried as described in Example 1.

### Example 3

### Preparation of monolithic melamine-formaldehyde aerogel

Resamine (commercial melamine-formaldehyde resin, Monsanto) is mixed in sufficient deionised distilled water to produce a solution containing ≤ 10% w/v of reactants. Hydrochloric acid is then added as a catalyst (until pH=2-3). The mixture is stirred to produce a homogeneous solution, the solution is poured into glass vials, sealed and allowed to cure to a gel at 85-95°C for seven days.

After curing is completed, the cross-linked gel is removed from the glass vial and treated as in Example 1 to yield a monolithic melamine-formaldehyde aerogel in the shape of a cylinder approx. 25mm x 80mm.

### Example 4

### Preparation of microspheres of melamine-formaldehyde aerogel

Resamine (commercial melamine-formaldehyde resin, Monsanto) is mixed in sufficient deionised distilled water to produce a solution containing ≤10% w/v of reactants. Hydrochloric acid is then added as a catalyst (until pH=2-3). The mixture is stirred to produce a homogenous solution.

Inverse emulsion polymerisation and work up as in Example 2 yields the title microspheres.

### Example 5

### General preparation of biodegradable organic aerogels

This preparation typically involves the condensation reaction of a multifunctional monomer with a linking agent (e.g. resorcinol with formaldehyde). Table 1 lists a variety of monomers and linking agents that can be used to prepare biodegradable organic aerogels. The preparation usually involves dissolving the monomer and linking agent in water (typically in a 1:2 molar ratio). After adding a catalyst (where appropriate), the mixture is stirred to produce a homogeneous solution. The solution is then cured under conditions to produce either monolithic or microspheric organic aerogels as described in Examples 1 and 2. After solvent exchange with acetone and then with liquid carbon dioxide these gels are then supercritically dried to produce the final biodegradable organic aerogel materials.

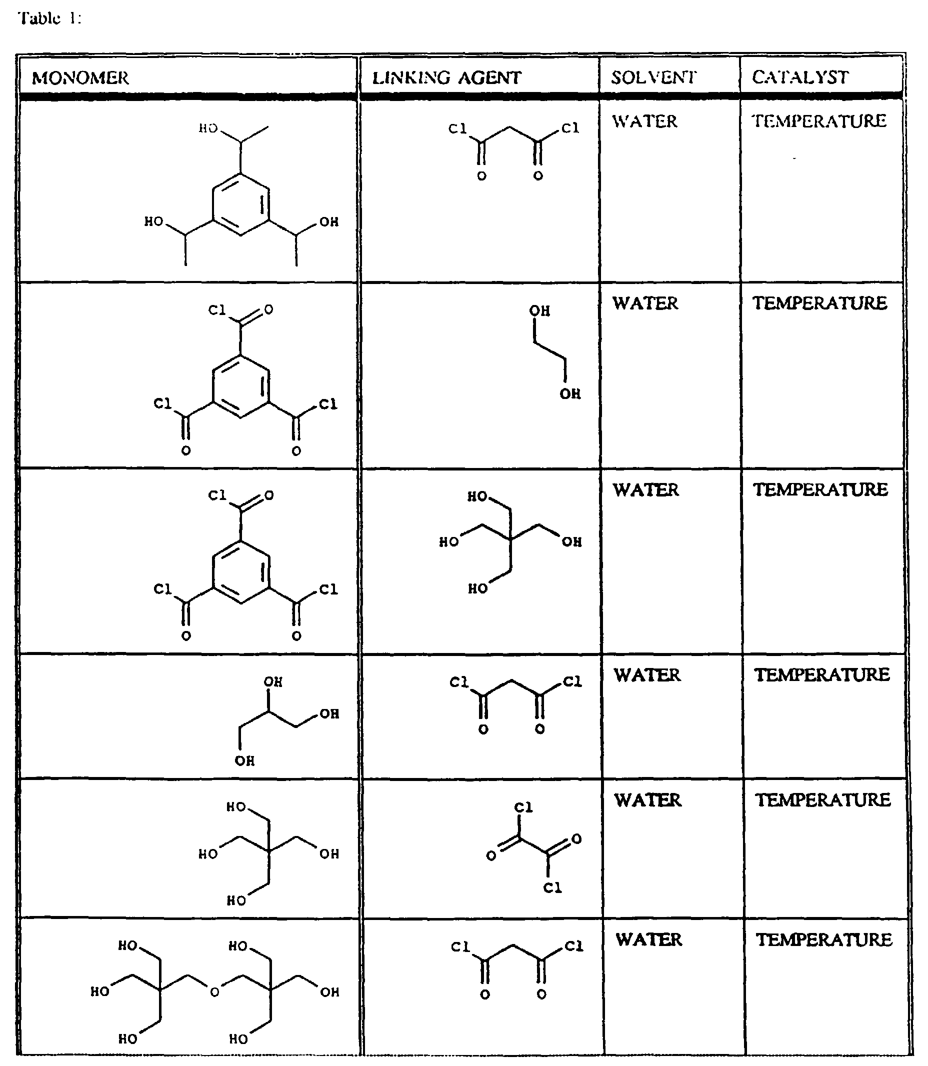

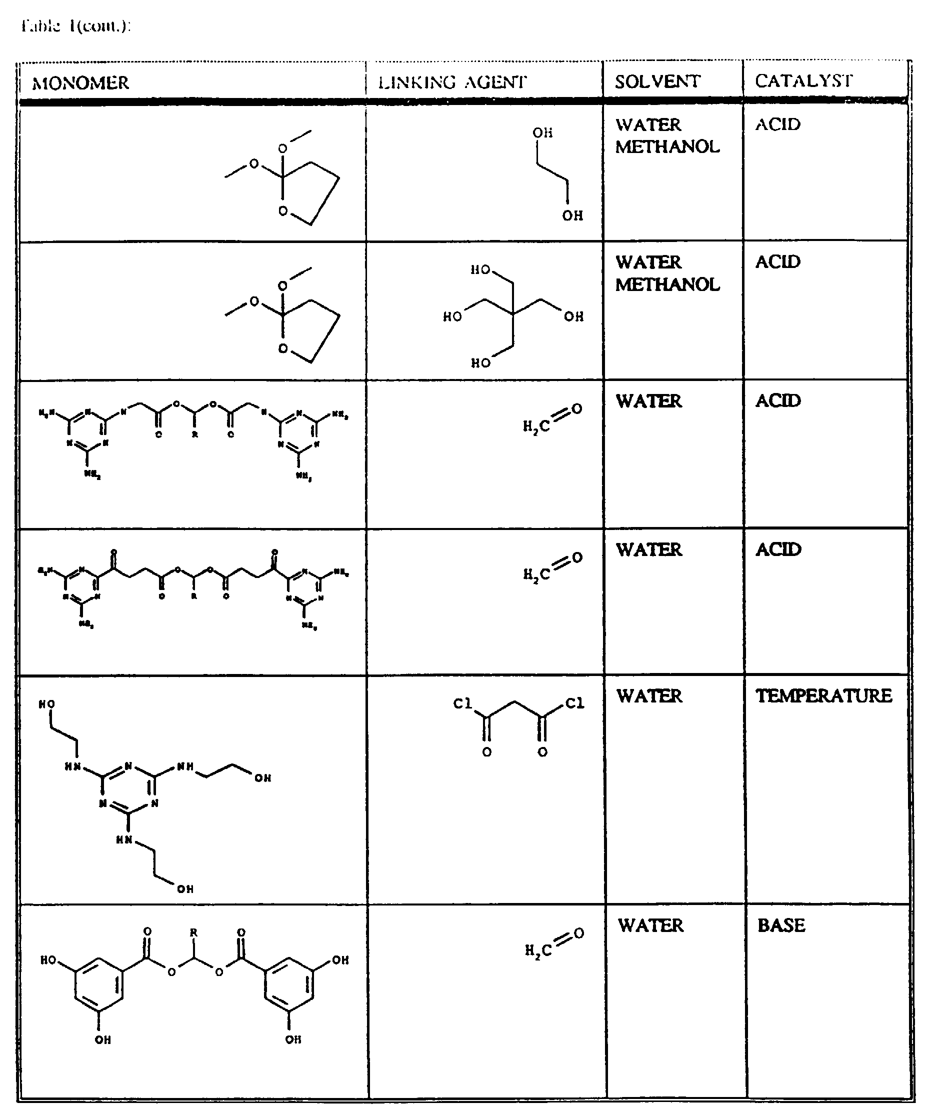

### EXAMPLES 6-10 - ULTRASOUND CONTRAST AGENTS

### Example 6

0.020 g carbonised aerogel, prepared by pyrolysis at 1050°C (as described in US-A-4873218 and US-A-5086085) of the aerogels of Examples 2 or 4 (microsphere diameter about 18µm), is mixed with 1 ml Pluronic F68 (2% in water). Microbubbles with diameter between 10 and 50µm (microscopic observation) are formed as air is released from the particles. The acoustic attenuation (dB/cm) is measured after dilution of 1 ml of the above aerogel suspension with 6 ml water.

### Example 7

To 0.020 g carbonised aerogel, prepared by pyrolysis at 1050°C (as described in US-A-4873218 and US-A-5086085) of the organic aerogels of Examples 2 or 4 (microsphere diameter about 18µm) is added 0.2g perfluoropentane at 25°C (below the boiling point of perfluoropentane). Pluronic F68 (2% in water, 1 ml) is added, and the mixture is heated to 60°C for 2 minutes. The aerogel particles start settling within 2-3 minutes, and gas is released from the particles as microbubbles (diameter between 10 and 100µm, microscopy). The acoustic attenuation (dB/cm) is measured after dilution of 1 ml of the above aerogel suspension with 6 ml water.

### Example 8

0.020 g carbonised aerogel, prepared by pyrolysis at 1050°C (as described in US-A-4873218 and US-A-5086085) of the organic aerogels of Examples 2 or 4 (microsphere diameter about 18µm), is pressurised with perfluorobutane (2 atm) for one hour and mixed with 1 ml of 2% Pluronic F68 in water. The aerogel particles become heavier than water and gas is released from the particles as microbubbles (diameter between 5 and 50µm, microscopy). The acoustic attenuation (dB/cm) is measured after dilution of 1 ml of the above aerogel suspension with 6 ml water.

### Example 9

0.050 g resorcinol-formaldehyde microspheres (from Example 2, particle diameter 10-35µm) is pressurised with perfluorobutane (2 atm) for one hour and mixed with 1 ml 2% Pluronic F68 in water. Gas is released from the particles as microbubbles (diameter between 5 and 100µm, microscopy). The acoustic attenuation (dB/cm) is measured after dilution of 1 ml of the above aerogel suspension with 6 ml water.

### Example 10

To 0.050 g melamine-formaldehyde microspheres (from Example 4, particle diameter 70-135µm) is added 0.5g perfluoropentane at 25°C (below the boiling point of perfluoropentane). Pluronic F68 (2% in water, 1 ml) is added, and the mixture is heated to 60°C for 2 minutes. Gas is released from the particles as microbubbles (diameter between 5 and 100µm, microscopy). The acoustic attenuation (dB/cm) is measured after dilution of 1 ml of the above aerogel suspension with 6 ml of water.

### EXAMPLES 11-15 - DRUG-LOADED AEROGELS

### Example 11

A cured monolithic or microsphere suspension is prepared from a resorcinol-formaldehyde solution (Examples 1 and 2). The cured gel is exchanged with acetone and supercritically dried from carbon dioxide to produce the dried organic aerogel. 0.050 g of the dried gel (in a 3 ml vessel connected with a tube) is exposed to ethanol vapour for at least 60 minutes. A saturated solution of testosterone adipate in ethanol is carefully added to the prewetted aerogel, and the drug is incorporated into the aerogel by gently rolling for 2 hours. Excess ethanol/drug is removed by pipetting, and a volume of ethanol is added to remove nonadsorbed drug by brief washing. After removal of the ethanol, the drug-loaded aerogel is carefully dried to produce the final testosterone adipate-incorporated organic aerogel.

### Example 12

A resorcinol-formaldehyde monolithic or microsphere aerogel is prepared and prewetted with ethanol vapor as described in Example 11. To 0.050 g of the prewetted aerogel, 1ml ethanol is carefully added, and the vial is rolled for at least 5 minutes. The ethanol is removed by pipetting, and replaced by 50% aqueous ethanol, and rolled for another 5 minutes. After a number of exchanges with decreasing concentrations of ethanol, the aerogel is suspended in water. A saturated aqueous solution of 5-fluorouracil is carefully added to the prewetted aerogel, and the aerogel is treated as described in Example 11 (with water as solvent) to produce a dry 5-fluorouracil-loaded organic aerogel.

### Example 13

### Drug introduction during gel synthesis

Resorcinol and formaldehyde (1:2 molar ratio) are mixed in sufficient deionised distilled water to produce a solution containing ≤10% w/v of reactants. Sodium carbonate is then added as a catalyst (resorcinol:catalyst molar ratio 50-400:1) and the mixture is then stirred to produce a homogeneous solution. 5-Fluorouracil is added to the solution until close to saturation; the solution is poured into glass vials, sealed, and allowed to cure to gel at 85-95°C for seven days. Further processing to prepare the final dried drug-incorporated organic aerogel (monolith or microsphere) proceeds as described in Examples 1 or 2.

### Example 14

### Drug introduction during solvent exchange

A resorcinol-formaldehyde solution is prepared as described in Example 1 or 2. After curing is complete, the cross-linked gel (monolith or microsphere) is exchanged with acetone saturated with testosterone adipate. The acetone/testosterone adipate-filled gel is placed in a jacketed pressure vessel (Polaron), the acetone is then exchanged with liquid carbon dioxide, and the gel is supercritically dried (Tc=31°C, Pc=7.4MPa) to produce the final dried drug-incorporated organic aerogel.

### Example 15

### Drug introduction during supercritical drying

A resorcinol-formaldehyde solution is prepared as described in Example 1 or 2. After curing is complete, the cross-linked gel (monolith or microsphere) is exchanged with acetone. The acetone-filled gel is placed in a jacketed pressure vessel (Polaron), the acetone is then exchanged with liquid carbon dioxide saturated with testosterone adipate, and the testosterone adipate-containing gel is supercritically dried (Tc=31°C; Pc=7.4 MPa) to produce the final dried drug-incorporated organic aerogel.

### EXAMPLES 16-18 - COATED AEROGELS

### Example 16

A drug-loaded resorcinol-formaldehyde aerogel (Example 12; 0.050 g) is carefully prewetted with ethanol as described (Example 11), and the solvent is gradually exchanged to water (Example 12). A solution of 5% human serum albumin (HSA) is added to the aerogel, and the vial is rolled for 60 minutes. Excess HSA solution is removed by pipetting and the HSA-coated aerogel is washed twice with a small amount of water. The aerogel is carefully dried to produce the drug-loaded, albumin-coated organic aerogel.

### Example 17

A drug-loaded resorcinol-formaldehyde aerogel is prepared (Example 12) and coated with HSA (Example 16). After removal of excess HSA solution, the HSA-coated aerogel is exposed to a solution of 0.25% aqueous glutaraldehyde for 60 minutes. Excess aldehyde is removed by pipetting and the aerogel coated with crosslinked HSA is washed twice with a small amount of water. The aerogel is carefully dried to produce the drug-loaded crosslinked albumin-coated organic aerogel.

### Example 18

A drug-loaded resorcinol-formaldehyde aerogel (Example 12; 0.050 g) is carefully prewetted with ethanol (Example 11), and the solvent is exchanged to water (Example 12). A solution of 1% chitosan in 1% acetic acid is carefully added to the aerogel, and the vial is rolled for 60 minutes. Excess chitosan is removed by pipetting, and the chitosan-coated aerogel is washed with a small volume of 0.05N sodium hydroxide, and finally washed twice with a small amount of water. The aerogel is carefully dried to produce the drug-loaded chitosan-coated organic aerogel.

### EXAMPLE 19 - ORGANIC AEROGELS AS IMPLANTS

The monomer/linker solution which ultimately forms the gel that is supercritically dried to an aerogel can be placed in a mould of practically any size or shape. After curing, the gel retains the shape of the mould and is then supercritically dried from carbon dioxide to produce the desired solid organic aerogel implant. Such implants can be used for both structural and drug-delivery applications.

Resorcinol and formaldehyde (1:2 molar ratio) are mixed in sufficient deionised distilled water to produce a solution containing ≤ 10% w/v of reactants. Sodium carbonate is then added as a catalyst (resorcinol:catalyst molar ratio 50-400:1). The mixture is stirred to produce a homogeneous solution, the solution is poured into glass cylinders (approx. 25mm x 80mm) or cast between glass plates separated by a polymer seal to produce a thin film (1-2cm on each side; ∼ 0.5mm thick). These moulds are sealed and allowed to cure to a gel at 85-95°C for seven days.

After curing is completed, the cross-linked gel is removed from the glass mould and the pore fluid (water) is exchanged with acetone. The acetone-filled gel is placed in a jacketed pressure vessel (Polaron), the acetone is then exchanged with liquid carbon dioxide, and the gel is supercritically dried (Tc=31°C, Pc=7.4 MPa). This procedure results in a monolithic resorcinol-formaldehyde organic aerogel retaining the shape of the glass mould (cylinder or thin film respectively) used to cure the original resorcinol/formaldehyde/carbonate mixture.

## Claims

1. A physiologically acceptable optionally carbonised organic aerogel for use in medicine, said aerogel having a porosity of 80-99.8% and being obtainable by supercritically drying a polymer gel.

2. A physiologically acceptable organic aerogel as claimed in claim 1 for use in a drug delivery system.

3. A physiologically acceptable organic aerogel as claimed in claim 2 which is in the form of a suspension of particles for injection.

4. A physiologically acceptable organic aerogel as claimed in claim 1 for use as a diagnostic agent.

5. A physiologically acceptable organic aerogel as claimed in claim 4 which is in the form of microparticles.

6. A physiologically acceptable organic aerogel as claimed in claim 5 for use as an ultrasound contrast agent.

7. A physiologically acceptable organic aerogel as claimed in claim 6 for use in echocardiography, said microparticles having sizes not exceeding 10 µm.

8. A physiologically acceptable organic aerogel as claimed in claim 6 for use in gastrointestinal imaging or investigations of the Fallopian tubes, said microparticles having sizes not exceeding 500 µm.

9. A physiologically acceptable organic aerogel as claimed in any of claims 6 to 8 wherein the microparticles contain gas selected from air, nitrogen, oxygen, hydrogen, nitrous oxide, carbon dioxide, helium, argon, sulphur hexafluoride and optionally fluorinated low molecular weight hydrocarbons.

10. A physiologically acceptable organic aerogel as claimed in claim 9 wherein the gas is a fluorinated alkane.

11. A physiologically acceptable organic aerogel as claimed in claim 10 wherein the gas is perfluorobutane or perfluoropentane.

12. A physiologically acceptable organic aerogel as claimed in claim 1 for use as artificial tissue, an organ or an organ component.

13. A physiologically acceptable organic aerogel as claimed in any of the preceding claims comprising a crosslinked polymer or a polymer obtained from at least one appropriate multifunctional monomer.

14. A physiologically acceptable organic aerogel as claimed in claim 13 wherein the polymer is biodegradable.

15. A physiologically acceptable organic aerogel as claimed in claim 13 or claim 14 wherein the polymer comprises a polyester or copolymer thereof, a polyorthoester or copolymer thereof, a non-peptide polyamide, a peptide polyamide, a polyanhydride, a polyphosphazene, a polyiminocarbonate, a polycyanoacrylate, hyaluronic acid, chitosan, chitin, collagen, gelatin, albumin or starch.

16. A physiologically acceptable organic aerogel as claimed in claim 14 wherein the polymer contains methylene diester groups of formula (I)
⁅CO-O-C(R¹R²)-O-CO⁆ (I)
where R¹ and R² each represent a hydrogen atom or a carbon-attached monovalent organic group or together form a carbon-attached divalent organic group.

17. A physiologically acceptable organic aerogel as claimed in any of the preceding claims which is surface-modified to affect absorption of proteins.

18. A physiologically acceptable organic aerogel as claimed in claim 17 which is surface-modified so as selectively to bind albumin.

19. A physiologically acceptable organic aerogel as claimed in any of claims 1 to 16 having a heparinised surface.

20. A physiologically acceptable organic aerogel as claimed in any of claims 1 to 16 the surface of which is sealed with a bioacceptable coating.

21. A process for the preparation of a physiologically acceptable organic aerogel as claimed in claim 1 which comprises supercritically drying a polymer gel and, if desired, pyrolysing the thus-obtained product.

22. A process as claimed in claim 21 wherein the polymer gel is supercritically dried from liquid carbon dioxide.

## Patentansprüche

1. Physiologisch akzeptables, gegebenenfalls carbonisiertes, organisches Aerogel zur medizinischen Verwendung, wobei das Aerogel eine Porosität von 80-99,8% aufweist und durch überkritisches Trocknen eines Polymergels erhältlich ist.

2. Physiologisch akzeptables, organisches Aerogel nach Anspruch 1 zur Verwendung in einem Wirkstoffabgabesystem.

3. Physiologisch akzeptables, organisches Aerogel nach Anspruch 2, das in Form einer Partikelsuspension zur Injektion vorliegt.

4. Physiologisch akzeptables, organisches Aerogel nach Anspruch 1 zur Verwendung als diagnostisches Mittel.

5. Physiologisch akzeptables, organisches Aerogel nach Anspruch 4, das in Form von Mikropartikeln vorliegt.

6. Physiologisch akzeptables, organisches Aerogel nach Anspruch 5 zur Verwendung als Ultraschallkontrastmittel.

7. Physiologisch akzeptables, organisches Aerogel nach Anspruch 6 zur Verwendung in der Echocardiographie, wobei die Mikropartikel eine Größe aufweisen, die 10 µm nicht übersteigt.

8. Physiologisch akzeptables, organisches Aerogel nach Anspruch 6 zur Verwendung für die gastrointestinale Bildgebung oder Untersuchungen der Eileiter, wobei die Mikropartikel eine Größe aufweisen, die 500 µm nicht übersteigt.

9. Physiologisch akzeptables, organisches Aerogel nach einem der Ansprüche 6 bis 8, worin die Mikropartikel ein Gas enthalten, das ausgewählt ist unter Luft, Stickstoff, Sauerstoff, Wasserstoff, Lachgas, Kohlendioxid, Helium, Argon, Schwefelhexafluorid und gegebenenfalls fluorierten Kohlenwasserstoffen niedrigen Molekulargewichts.

10. Physiologisch akzeptables, organisches Aerogel nach Anspruch 9, worin das Gas ein fluoriertes Alkan ist.

11. Physiologisch akzeptables, organisches Aerogel nach Anspruch 10, worin das Gas Perfluorbutan oder Perfluorpentan ist.

12. Physiologisch akzeptables, organisches Aerogel nach Anspruch 1 zur Verwendung als künstliches Gewebe, Organ oder Organkomponente.

13. Physiologisch akzeptables, organisches Aerogel nach einem der vorhergehenden Ansprüche, das ein quervernetztes Polymer oder ein aus wenigstens einem geeigneten multifunktionalen Monomer erhaltenes Polymer umfaßt.

14. Physiologisch akzeptables, organisches Aerogel nach Anspruch 13, worin das Polymer biologisch abbaubar ist.

15. Physiologisch akzeptables, organisches Aerogel nach Anspruch 13 oder 14, worin das Polymer einen Polyester oder ein Copolymer davon, einen Polyorthoester oder ein Copolymer davon, ein nicht-peptidisches Polyamid, ein Peptid-Polyamid, ein Polyanhydrid, ein Polyphosphazen, ein Polyiminocarbonat, ein Polycyanoacrylat, Hyaluronsäure, Chitosan, Chitin, Collagen, Gelatine, Albumin oder Stärke umfaßt.

16. Physiologisch akzeptables, organisches Aerogel nach Anspruch 14, worin das Polymer Methylendiestergruppen der Formel (I)
⁅ CO-O-C(R¹R²) -O-CO ⁆ (I)
enthält,
worin R¹ und R² jeweils für ein Wasserstoffatom oder eine kohlenstoffgebundene, monovalente organische Gruppe stehen oder zusammen eine kohlenstoffgebundene, divalente organische Gruppe bilden.

17. Physiologisch akzeptables, organisches Aerogel nach einem der vorhergehenden Ansprüche, das oberflächenmodifiziert ist, um die Absorption von Proteinen zu beeinflussen.

18. Physiologisch akzeptables, organisches Aerogel nach einem der Ansprüche 17, das oberflächenmodifiziert ist, um selektiv Albumin zu binden.

19. Physiologisch akzeptables, organisches Aerogel nach einen der Ansprüche 1 bis 16 mit einer heparinisierten Oberfläche.

20. Physiologisch akzeptables, organisches Aerogel nach einem der Ansprüche 1 bis 16, dessen Oberfläche mit einer biologisch akzeptablen Beschichtung verriegelt ist.

21. Verfahren zur Herstellung eines physiologisch akzeptablen, organischen Aerogels nach Anspruch 1, wobei man ein Polymergel überkritisch trocknet und erwünschtenfalls das so erhaltene Produkt pyrolisiert.

22. Verfahren nach Anspruch 21, worin das Polymergel überkritisch aus flüssigem Kohlendioxid getrocknet wird.

## Revendications

1. Aérogel organique physiologiquement acceptable, éventuellement carbonisé, pour une utilisation en médecine, ledit aérogel ayant une porosité de 80 à 99,8% et pouvant être obtenu par séchage supercritique d'un gel de polymères.

2. Aérogel organique physiologiquement acceptable selon la revendication 1, pour une utilisation dans un système de délivrance de médicaments.

3. Aérogel organique physiologiquement acceptable selon la revendication 2, qui se présente sous la forme d'une suspension de particules pour injection.

4. Aérogel organique physiologiquement acceptable selon la revendication 1, pour une utilisation en tant qu'agent diagnostique.

5. Aérogel organique physiologiquement acceptable selon la revendication 4, qui se présente sous la forme de microparticules.

6. Aérogel organique physiologiquement acceptable selon la revendication 5, pour une utilisation en tant qu'agent de contraste ultrasonique.

7. Aérogel organique physiologiquement acceptable selon la revendication 6, pour une utilisation en échocardiographie, lesdites microparticules ayant une taille qui ne dépasse pas 10 µm.

8. Aérogel organique physiologiquement acceptable selon la revendication 6, pour une utilisation en imagerie gastro-intestinale ou pour l'examen des trompes de Fallope, lesdites microparticules ayant une taille qui ne dépasse pas 500 µm.

9. Aérogel organique physiologiquement acceptable selon l'une quelconque des revendications 6 à 8, dans lequel les microparticules contiennent un gaz choisi parmi l'air, l'azote, l'oxygène, l'hydrogène, l'oxyde nitreux, le dioxyde de carbone, l'hélium, l'argon, l'hexafluorure de soufre et éventuellement des hydrocarbures fluorés de bas poids moléculaire.

10. Aérogel organique physiologiquement acceptable selon la revendication 9, dans lequel le gaz est un alcane fluoré.

11. Aérogel organique physiologiquement acceptable selon la revendication 10, dans lequel le gaz est le perfluorobutane ou le perfluoropentane.

12. Aérogel organique physiologiquement acceptable selon la revendication 1, pour une utilisation comme tissu artificiel, organe ou composant d'organe.

13. Aérogel organique physiologiquement acceptable selon l'une quelconque des revendications précédentes, comprenant un polymère réticulé ou un polymère obtenu à partir d'au moins un monomère multifonctionnel approprié.

14. Aérogel organique physiologiquement acceptable selon la revendication 13, dans lequel le polymère est biodégradable.

15. Aérogel organique physiologiquement acceptable selon la revendication 13 ou la revendication 14, dans lequel le polymère comprend un polyester ou un copolymère de ce dernier, un polyorthoester ou un copolymère de ce dernier, un polyamide non peptidique, un polyamide peptidique, un polyanhydride, un polyphosphazène, un polyiminocarbonate, un polycyanoacrylate, de l'acide hyaluronique, du chitosane, de la chitine, du collagène, de la gélatine, de l'albumine ou de l'amidon.

16. Aérogel organique physiologiquement acceptable selon la revendication 14, dans lequel le polymère contient des groupes méthylène diester de formule (I)
⁅CO-O-C(R¹R²)-O-CO⁆ (I)
dans laquelle R¹ et R² représentent chacun un atome d'hydrogène ou un groupe organique monovalent lié à un carbone ou bien forment ensemble un groupe organique divalent lié à un carbone.

17. Aérogel organique physiologiquement acceptable selon l'une quelconque des revendications précédentes, qui est modifié en surface afin d'influer sur l'absorption des protéines.

18. Aérogel organique physiologiquement acceptable selon la revendication 17, qui est modifié en surface de manière à se lier sélectivement à l'albumine.

19. Aérogel organique physiologiquement acceptable selon l'une quelconque des revendications 1 à 16, ayant une surface héparinée.

20. Aérogel organique physiologiquement acceptable selon l'une quelconque des revendications 1 à 16, dont la surface est obturée avec un enrobage biologiquement acceptable.

21. Procédé de préparation d'un aérogel organique physiologiquement acceptable selon la revendication 1, comprenant le séchage supercritique d'un gel de polymères et, si nécessaire, la pyrolyse du produit ainsi obtenu.

22. Procédé selon la revendication 21, dans lequel le gel de polymères est soumis à un séchage supercritique à partir de dioxyde de carbone liquide.
